**Europäisches Patentamt**

**European Patent Office**

**Office européen des brevets**

(19)

(11) Veröffentlichungsnummer : **0 355 323 B1**

## (12) EUROPÄISCHE PATENTSCHRIFT

(45) Veröffentlichungstag der Patentschrift :
**25.03.92 Patentblatt 92/13**

(51) Int. Cl.⁵ : **A61F 2/24**

(21) Anmeldenummer : **89111662.6**

(22) Anmeldetag : **27.06.89**

(54) **Herzklappenprothese.**

(30) Priorität : **25.08.88 DE 3828781**

(43) Veröffentlichungstag der Anmeldung :
**28.02.90 Patentblatt 90/09**

(45) Bekanntmachung des Hinweises auf die
Patenterteilung :
**25.03.92 Patentblatt 92/13**

(84) Benannte Vertragsstaaten :
**AT BE CH DE ES FR GB GR IT LI LU NL SE**

(56) Entgegenhaltungen :
**EP-A- 0 113 681**
**EP-A- 0 121 473**

(73) Patentinhaber : **B. Braun Melsungen AG**
**Carl-Braun Strasse**
**W-3508 Melsungen (DE)**

(72) Erfinder : **Knoch, Martin, Dr.-Ing.**
**Kullenhofwinkel 34**
**W-5100 Aachen (DE)**
Erfinder : **Reul, Helmut, Prof. Dr.-Ing.**
**Akazienstrasse 65**
**W-5160 Düren (DE)**
Erfinder : **Rau, Günter, Prof. Dr. rer.nat.**
**Fuchserde 50**
**W-5100 Aachen (DE)**

(74) Vertreter : **Selting, Günther, Dipl.-Ing. et al**
**Patentanwälte von Kreisler, Selting, Werner**
**Deichmannhaus am Hauptbahnhof**
**W-5000 Köln 1 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Herzklappenprothese nach dem Oberbegriff des Patentanspruchs 1.

Die natürlichen Herzklappen sind drei- bzw. zweiflügelige Segelklappen, die technisch gesprochen die Funktion von Rückschlagventilen haben, welche das Blut in einer Richtung durchlassen, in Gegenrichtung jedoch sperren. Beim Ersatz der natürlichen Herzklappen durch mechanische Pendel- oder Kippscheibenprothesen werden einflügelige oder zweiflügelige Klappen eingesetzt, bei denen klappenförmige Schließkörper in einem Klappenring, der an der betreffenden Öffnung des Herzens festgenäht wird, durch den Blutdruck bzw. die Blutströmung bewegbar sind. Beim Langzeiteinsatz solcher Herzklappenprothesen können sich schwerwiegende Probleme ergeben, die eine lebenslange Einnahme von Antikoagulantien durch den Patienten oder das Auswechseln der Prothese erforderlich machen. Beispielsweise besteht die Gefahr, daß am Klappenring oder an den Halteeinrichtungen des Schließkörpers Thromben gebildet werden, die die Beweglichkeit des Schließkörpers und die Dichtheit der Herzklappenprothese beeinträchtigen. Außerdem kann Körpergewebe in den Strömungsbereich des Blutes einwachsen. Die Gefahr der Thrombenbildung besteht insbesondere an den in Ausnehmungen des Klappenrings eingreifenden Lagerzapfen des Schließkörpers. Daher müssen besondere Vorkehrungen getroffen werden, um eine Umspülung der Lagerzapfen und eine Durchspülung der die Lagerzapfen haltenden Ausnehmungen mit dem strömenden Blut sicherzustellen.

DE 37 01 755 C1 beschreibt eine Herzklappenprothese, bei der die Lagerzapfen des Klappenrings in Gelenkpfannen gelagert sind, durch die mindestens ein Spülkanal hindurchführt. Eine solche Gelenkpfannenstruktur ist einerseits schwierig herzustellen und sie liefert andererseits nicht die nötige Beweglichkeit für den Schließkörper.

Eine Herzklappenprothese, von der der Oberbegriff des Patentanspruchs 1 ausgeht, ist aus EP 0 113 681 A1 bekannt. Bei dieser Herzklappenprothese sind in der Wand des Klappenringes Ausnehmungen vorgesehen, deren Querschnitt größer ist als derjenige der Lagerzapfen des Schließkörpers, so daß die Lagerzapfen bei der Öffnungs- und Schließbewegung des Schließkörpers zusätzlich zur Schwenkbewegung eine translatorische Bewegung ausführen. Die Ausnehmungen sind Mulden oder Sacklöcher, wobei die stirnseitigen Enden der Lagerzapfen bei der Bewegung des Klappenringes den Grund der Ausnehmungen nach Art eines Scheibenwischers überstreichen. Dadurch entsteht bei jeder Schließkörperbewegung eine Abstreifwirkung. Nachteilig ist jedoch, daß nicht alle Flächenteile von der Abstreifung gleichmäßig erfaßt werden und daß in den Ausnehmungen, insbesondere an deren Rändern, Bereiche entstehen, in denen sich Thromben bilden können.

Bei den bekannten Herzklappenprothesen entstehen an den Lagerstellen des Schließkörpers Rezirkulationsströmungen und Totwassergebiete mit der Folge eines ungenügenden Auswaschens der Berührungsstellen zwischen Klappenring und Schließkörper. Durch dort anhaftende Thromben besteht die Gefahr des unvollständigen Öffnens und Schließens.

Der Erfindung liegt die Aufgabe zugrunde, eine Herzklappenprothese der im Oberbegriff des Patentanspruchs 1 angegebenen Art zu schaffen, bei der Thrombenbildungen in dem hierfür besonders anfälligen Bereich der Lagerstellen vermieden werden.

Die Lösung dieser Aufgabe erfolgt erfindungsgemäß mit dem im kennzeichnenden Teil des Patentanspruchs 1 angegebenen Merkmal.

Bei der erfindungsgemäßen Herzklappenprothese sind die Ausnehmungen, in denen der Schließkörper gelagert ist, Durchgangslöcher. Da der Querschnitt dieser Durchgangslöcher größer ist als derjenige der Lagerzapfen, werden Durchströmkanäle gebildet, so daß ein geringer Teil des in den Klappenring einströmenden Blutes durch die Durchgangslöcher seitlich austritt und dabei die Lagerzapfen umströmt. Dieses Umströmen tritt beispielsweise beim Aortenklappenersatz nicht nur während der Systole auf, wenn der Schließkörper sich in der Öffnungsstellung befindet, sondern in geringfügigem Maße auch während der Diastole, wenn der Schließkörper sich in der Schließstellung befindet. Die Lagerzapfen werden also in beiden Endstellungen umspült, so daß die jeweils für die Umspülung freiliegenden Bereiche der lagerzapfen ständig wechseln.

Die Erfindung eignet sich insbesondere für einflügelige Herzklappenprothesen, ist aber auch bei der Lagerung der Schließkörper von zweiflügeligen Herzklappenprothesen anwendbar. Die Übergangsflächen sowohl vom Schließkörper als auch vom Klappenring in die entsprechenden Lagerelemente müssen so ausgebildet sein, daß bei der Durchströmung eine möglichst gleichförmige Verteilung der Wandschubspannungen an den Klappenoberflächen vorliegt. Gleichzeitig sollte die Versperrung des Ringquerschnitts im geöffneten Zustand der Klappe minimal sein. Die Lagerstrukturen dürfen keinen wesentlichen Einfluß auf die Druckverteilung an der Schließkörperoberfläche ausüben. Diese Bedingung ist in der Regel erfüllt, wenn keine Strömungsablösungen hervorgerufen werden.

Gemäß Anspruch 3 verläuft der Durchlaß des Schließkörpers schräg zur Normalen der Einlaßöffnung. Durch einen solchen schrägen Durchlaß wird die durch den geöffneten Schließkörper bewirkte Strömungsablenkung unterstützt. Die Langlöcher verlaufen parallel zum Durchlaß. Dadurch werden Klemmungen des Ran-

EP 0 355 323 B1

des des Schließkörpers am Klappenring beim Öffnen vermieden. Die Länge des Klappenringes kann vermindert werden.

Die Lagerzapfen müssen nicht notwendigerweise rund oder zylindrisch sein. Dies ist insbesondere deshalb nicht erforderlich, weil der Schwenkwinkel des Schließkörpers beschränkt ist. Nur mindestens über diesen Schwenkwinkel von etwa 70° ist eine zylindrische Stützfläche wünschenswert. Es können daher auch langgestreckte Lagerzapfenquerschnitte verwendet werden, die nur an einem Ende eine Rundung aufweisen. Es ist auch nicht unbedingt erforderlich, daß die Durchgangslöcher Langlöcher sind. Alternativ können die Durchgangslöcher auch die Form eines Kreissektors haben. Wichtig ist nur, daß die Lagerzapfen in den Durchgangslöchern ein Spiel haben und daß während des Übergangs von der Öffnungsstellung zur Schließstellung jeweils andere Umfangsbereiche der Lagerzapfen in den Durchgangslöchern freiliegen.

Im folgenden wird unter Bezugnahme auf die Zeichnungen ein Ausführungsbeispiel der Erfindung näher erläutert.

Es zeigen:

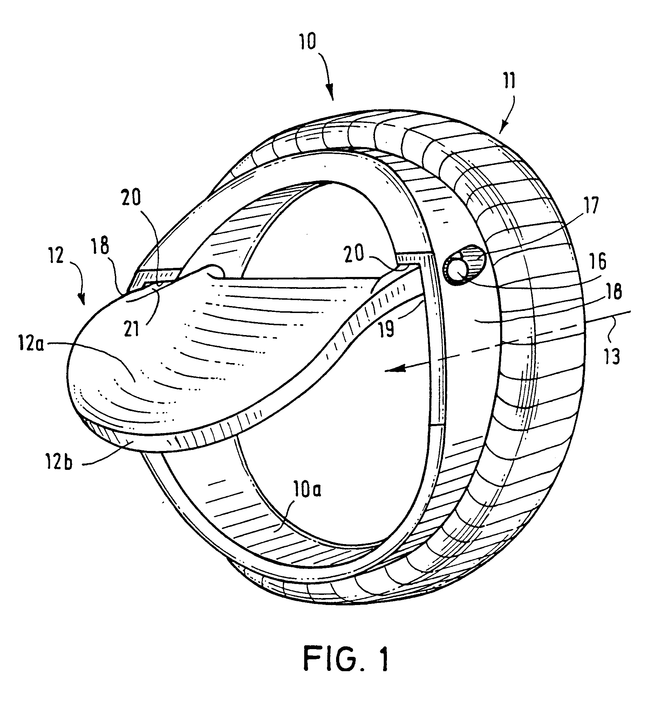

Fig. 1 eine perspektivische Ansicht der Herzklappenprothese,

Fig. 2 einen Längsschnitt durch die Herzklappenprothese im geöffneten Zustand,

Fig. 3 eine Draufsicht von Fig. 2,

Fig. 4 einen Schnitt durch eine Lagerstelle entlang der Linie IV-IV von Fig. 2,

Fig. 5 eine perspektivische Ansicht der Lagerstelle ohne eingesetzten Schließkörper, und

Fig. 6 eine schematische Darstellung des Einsetzens des Schließkörpers in den Klappenring.

Die als Ausführungsbeispiel dargestellte einflügelige Herzklappenprothese weist einen im wesentlichen kreisförmigen Klappenring 10 auf. Am Umfang des Klappenringes 10 ist ein Nahtring 11 zum Befestigen der Herzklappe am Körpergewebe angebracht. Am Klappenring 10 ist der klappenförmige Schließkörper 12 um eine quer zur Ringachse verlaufende Achse schwenkbar gelagert. Diese Schwenkachse befindet sich im Abstand von der Ringachse, so daß der Schließkörper 12 durch den in Richtung des Pfeiles 13 wirkenden Blutdruck geöffnet werden kann, während er durch einen in Gegenrichtung wirkenden Blutdruck in die Schließstellung gebracht wird.

Die Innenfläche 10a des Klappenringes 10 bildet einen etwa zylindrischen Durchlaß, dessen Achse 14 unter einem Winkel a zur Normalen der Einlaßöffnung 15 des Klappenringes verläuft. Die Achse 14 des von der Innenfläche 10a gebildeten Durchlasses verläuft annähernd parallel zu dem geöffneten Schließkörper 12.

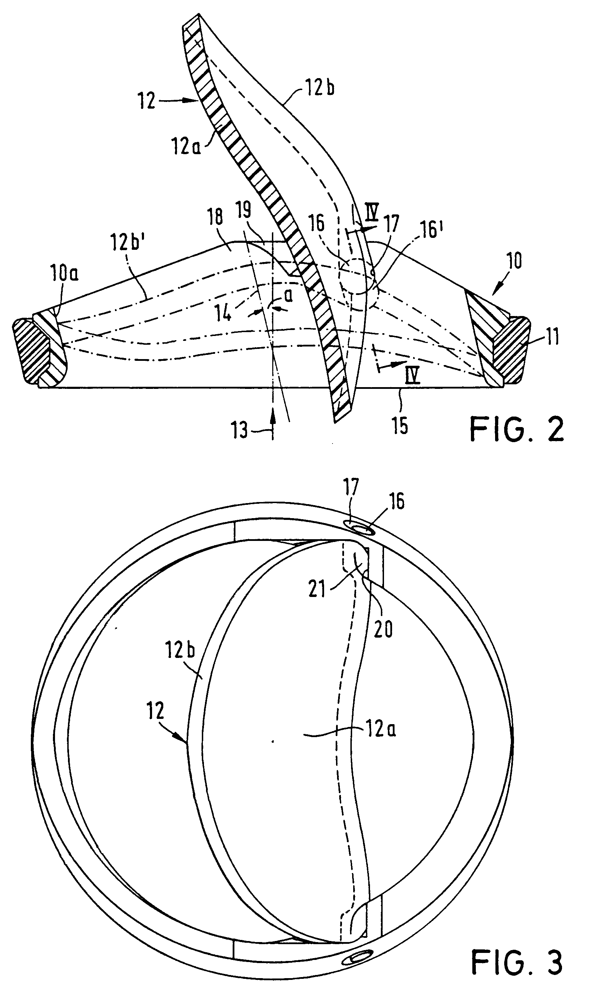

Wie Fig. 2 zeigt, ist die Skelettlinie des schraffiert dargestellten Schließkörperquerschnitts leicht S-förmig gekrümmt. Die Skelettlinie liegt in der quer zur Lagerachse verlaufenden Mittelebene des Schließkörpers. Zusätzlich ist der Schließkörper 12 in Querrichtung gewölbt, d.h. sein Mittelbereich 12a steht gegen die durch den Pfeil 13 bezeichnete Durchflußrichtung weiter vor als der Randbereich 12b.

An dem Umfang des Schließkörpers 12 sind zweinach entgegengesetzten Richtungen abstehende Lagerzapfen 16 angeordnet, deren gemeinsame Achse die Schwenkachse des Schließkörpers bildet. Diese Lagerzapfen 16 sitzen jeweils in einem durch die Wand des Klappenringes 10 hindurchgehenden Durchgangsloch 17. Die Lagerzapfen 16 sind bei diesem Ausführungsbeispiel zylindrisch. Die Breite der Durchgangslöcher ist nur geringfügig größer als der Durchmesser der Lagerzapfen 16, so daß die Lagerzapfen 16 sich ohne Klemmung in den Duchgangslöchern 17 drehen können. Die Durchgangslöcher 17 sind als Langlöcher ausgebildet, wobei die Länge etwa doppelt so groß ist wie der Durchmesser der Lagerzapfen 16.

Da der Nahtring 11 den Klappenring 10 auf seinem gesamten Umfang umgibt und da die Durchgangslöcher 17 nicht von dem Nahtring 11 bedeckt sein dürfen, weist der Klappenring an seinem stromabwärts gerichteten Ende zwei gegenüberliegende Laschen 18 auf, in deren Bereich die Durchgangsöffnungen 17 derart angeordnet sind, daß sie nicht von dem Nahtring 11 bedeckt werden.

Die als Langlöcher ausgebildeten Durchgangsöffnungen 17 verlaufen parallel zu der Achse 14 des Ringdurchlasses. In der Öffnungsstellung des Schließkörpers 12 werden die Lagerzapfen 16 gegen das stromabwärts gerichtete Ende 17a des Durchgangsloches 17 gedrückt, während sie bei geschlossenem Schließkörper gegen das stromaufwärts gerichtete Ende 17b gedrückt werden. Der Schließkörper führt also zusätzlich zu der rotatorischen Bewegung um die Achse der Lagerzapfen 16 auch eine translatorische Bewegung in Richtung der Langlöcher aus. Die Enden 17a und 17b der Durchgangslöcher sind so abgerundet, daß an ihnen eine vollflächige Abstützung des anliegenden Bereichs der Umfangsfläche des Lagerzapfens 16 erfolgt, so daß die Flächenpressungen gering gehalten werden.

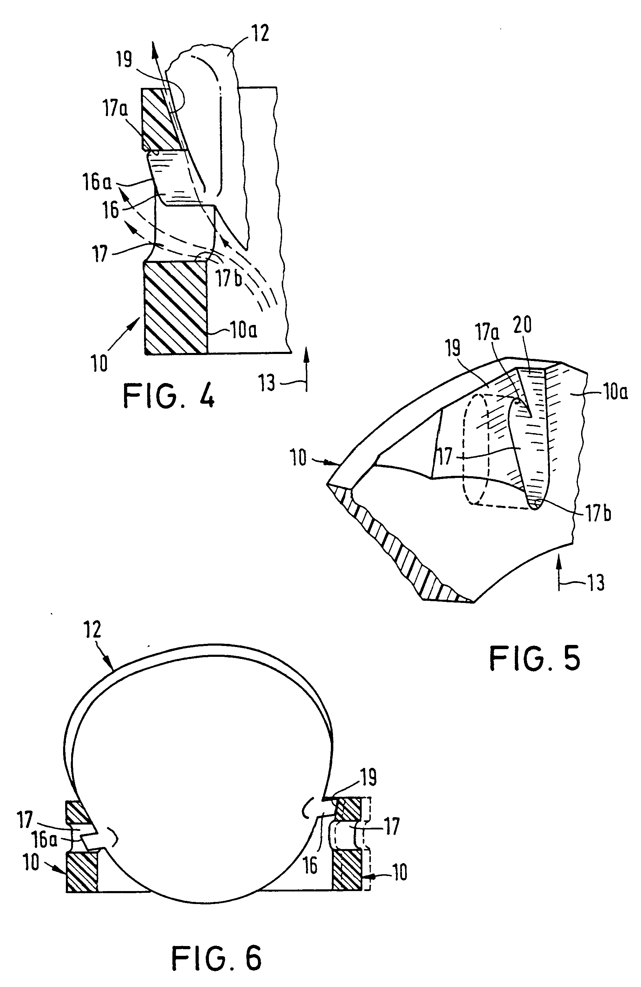

Wie Fig. 4 zeigt, haben die Lagerzapfen 16 jeweils schräge Stirnflächen 16a, wobei die Lagerzapfen jedoch in den Durchgangsöffnungen 17 vollständig versenkt sind. Dies bedeutet, daß die Lagerzapfen 16 nicht über die Außenkontur des Klappenringes 10 hinausragen.

Der Klappenring 10 ist gemäß Fig. 5 an jeder Lagerstelle mit einer an seiner Innenseite vorgesehenen Aussparung 19 versehen, die durch eine tangential zu dem Langloch 17 verlaufende Anschlagfläche 20 begrenzt ist. Diese Ausrichtung der Anschlagfläche 20 gewährleistet, daß der Schließkörper 12 unabhängig von der Position der Lagerzapfen 16 in den Durchgangsöffnungen 17 immer den gleichen Öffnungswinkel einnimmt. Die Aussparung 19 hat ihre größte Tiefe am stromabseitigen Rand des Klappenringes 10 und ihre Tiefe verringert sich stromaufwärts. Beim Einsetzen des Schließkörpers 12 in den Klappenring 10 von der Abströmseite her, wird zunächst der eine Lagerzapfen 16 in die entsprechende Durchgangsöffnung 17 eingesetzt. Dann wird der andere Lagerzapfen 16 mit seiner Schrägfläche 16a gemäß Fig. 6 gegen die Aussparung 19 gesetzt. Beim Vorschieben des Schließkörpers 12 erfolgt eine geringfügige radiale Verformung des Klappenringes 10, was in Fig. 6 durch die gestrichelten Linien angedeutet ist. Die Schrägfläche 16a des Lagerzapfens 16 gleitet vollflächig an der Schrägfläche der Ausnehmung 19, bis der Lagerzapfen 16 in dem Durchgangsloch 17 einrastet. Durch die aufeinander gleitenden Schrägflächen des Lagerzapfens 16 und der Aussparung 19 werden bei der Schließkörpermontage Beschädigungen an Schließkörper und Klappenring vermieden.

An der stromabwärts gerichteten Rückseite des Schließkörpers 12 sind angrenzend an die Lagerzapfen 16 abgeflachte Anschlagflächen 21 (Fig. 3) vorgesehen, die sich in der Öffnungsstellung des Schließkörpers flach gegen die Anschlagflächen 20 anlegen und somit die Öffnungsstellung begrenzen. Die Schließstellung des Schließkörpers wird dadurch begrenzt, daß der Rand 12b gegen die Innenseite 10a des Klappenrings 10 stößt.

In Fig. 2 ist der Schließkörper in der geöffneten Stellung in durchgezogenen Linien dargestellt. Der Verlauf des Randes 12b des Schließkörpers 12 im geschlossenen Zustand ist gestrichelt dargestellt und mit 12b′ bezeichnet. Im Schließzustand nehmen die Lagerzapfen in den Durchgangslöchern 17 die mit 16′ bezeichnete Position ein, d.h. sie stützen sich an den stromaufwärts gerichteten Enden 17b der Langlöcher ab.

In Fig. 4 ist der Verlauf der Blutströmung im Öffnungszustand des Schließkörpers 12 an der Lagerstelle dargestellt. Man erkennt, daß ein großer Teil der Strömung durch das Durchgangsloch 17 nach außen austritt und dabei den Zapfen 16 umströmt, und daß ein Teil der Strömung den Spalt zwischen dem Rand des Schließkörpers 12 und der Aussparung 19 durchspült.

## Patentansprüche

1. Herzklappenprothese mit einem Klappenring (10), in dem mindestens ein Schließkörper (12) schwenkbar gelagert ist, mit vom Schließkörper abstehenden Lagerzapfen (16), die in Ausnehmungen an der Innenseite des Klappenringes (10) eingreifen, wobei die Ausnehmungen größeren Querschnitt haben als die Lagerzapfen (16), derart, daß die Lagerzapfen bei der Schwenkbewegung des Schließkörpers (12) unterschiedliche Bereiche der Ausnehmungen ausfüllen, **dadurch gekennzeichnet,** daß die Ausnehmungen Durchgangslöcher (17) sind, die zur Außenseite des Klappenringes (10) hin offen sind.

2. Herzklappenprothese nach Anspruch 1, dadurch gekennzeichnet, daß die Durchgangslöcher (17) Langlöcher sind, deren Länge eine Verschiebung der Lagerzapfen zuläßt.

3. Herzklappenprothese nach Anspruch 1 oder 2, dadurch gekennzeichnet, daß die Innenfläche (10a) des Klappenrings (10) einen Durchlaß bildet, dessen Achse (14) unter einem Winkel (a) schräg zu der Normalen der Einlaßöffnung (15) des Klappenrings (10) verläuft, wobei der Winkel (a) der Schrägstellung in einer rechtwinklig zur Achse der Lagerzapfen (16) verlaufenden Ebene liegt, derart, daß die Achse (14) des Durchlasses annähernd parallel zu dem geöffneten Schließkörper (12) verläuft.

4. Herzklappenprothese nach den Ansprüchen 2 und 3, dadurch gekennzeichnet, daß die Langlöcher parallel zur Achse (14) des Durchlasses verlaufen.

5. Herzklappenprothese nach einem der Ansprüche 1 bis 4, dadurch gekennzeichnet, daß der Schließkörper (12) an seiner stromab liegenden Seite angrenzend an die Lagerzapfen (16) Anschlagflächen (21) aufweist, die mit Anschlagflächen (20) des Klappenringes (10) zusammenwirken und die Öffnungsbewegung des Schließkörpers (12) auf einen Winkel unter 80° begrenzen.

6. Herzklappenprothese nach einem der Ansprüche 1 bis 5, dadurch gekennzeichnet, daß die Lagerzapfen (16) nicht über die Außenkontur des Klappenringes (10) vorstehen und schräge Stirnflächen (16a) aufweisen, die in der Öffnungsstellung des Schließkörpers (12) stromaufwärts zurückweichen.

7. Herzklappenprothese nach Anspruch 6, dadurch gekennzeichnet, daß an der Innenseite des Klappenringes (10), angrenzend an die Durchgangslöcher (17), Schrägflächen (19) vorgesehen sind, die in Durchströmrichtung nach außen zurückweichen und an Anschlagflächen für den Schließkörper (12) angrenzen.

8. Herzklappenprothese nach einem der Ansprüche 1 bis 7, dadurch gekennzeichnet, daß der Schließkörper (12) eine S-förmig gekrümmte Skelettlinie hat und zusätzlich in Querrichtung gewölbt ist.

## Claims

1. A cardiac valve prosthesis having a valve ring (10) in which at least one closing body (12) is supported to be swiveled, and journal pins (16) projecting from the closing body and engaging into recesses at the inner surface of the valve ring (10), said recesses being larger in diameter than the journal pins (16) in such a manner that the journal pins, when the closing body (12) is swiveled, fill different areas of the recesses, **characterized in** that the recesses are through holes (17) being open towrards the exterior of the valve ring (10).

2. A cardiac valve prosthesis according to claim 1, characterized in that the through holes (17) are longitudinal holes having such a length that the journal pins (16) can be shifted within the through holes.

3. A cardiac valve prosthesis according to claim 1 or 2, characterized in that the inner surface (10a) of the valve ring (10) forms a passage having its axis (14) extending under an angle (a) obliquely to the normal line of the inlet opening (15) of the valve ring (10), the angle (a) of said oblique position being arranged in a plane extending at a right angle to the axis of the journal pin (16) such that the axis (14) of the passage extends substantially in parallel to the opened closing body (12).

4. A cardiac valve prosthesis according to claims 2 and 3, characterized in that the longitudinal holes extend parallel to the axis (14) of the passage.

5. A cardiac valve prosthesis according to one of claims 1 to 4, characterized in that the closing body (12), at its downstream end and adjacent to the journal pins (16), is provided with abutting faces (21) which cooperate with abutting faces (20) of the valve ring (10) and limit the opening movement of the closing body (12) to an angle smaller than 80°.

6. A cardiac valve prosthesis according to one of claims 1 to 5, characterized in that the journal pins (16) do not project beyond the outer contour of the valve ring (10) and have oblique end faces (16a) which, in the opening position of the closing body (12), recede in upstream direction.

7. A cardiac valve prosthesis according to claim 6, characterized in that oblique surfaces (19) are provided at the inner surface of the valve ring (10) and adjacent to the through holes (17), which oblique surfaces (19) recede in flow direction towards the exterior and adjoin abutting surfaces for the closing body (12)

8. A cardiac valve prosthesis according to one of claims 1 to 7, characterized in that the closing body (12) has its skeleton line curved in the manner of an S and is also curved in transverse direction.

## Revendications

1. Prothèse de valve cardiaque comportant une bague (10) de valve dans laquelle est logé à pivotement au moins un corps de fermeture (12), comportant des tourillons (16) à distance d' corps de fermeture qui pénètrent dans des évidements sur le côté intérieur de la bague (10) de valve, les évidements étant d'une section transversale supérieure à celle des tourillons (16) de telle manière que les tourillons remplissent, lors du mouvement de pivotement du corps de fermeture (12), des zones différentes des évidements, **caractérisée en ce que** les évidements sont des trous traversants (17) qui sont ouverts vers le côté extérieur de la bague (10) de valve.

2. Prothèse de valve cardiaque selon la revendication 1, caractèrisée en ce que les trous traversants (17) sont des trous longitudinaux dont la longueur permet un coulissement des tourillons.

3. Prothèse de valve cardiaque selon la revendication 1 ou 2, caractérisée en ce que la surface intérieure (10a) de la bague (10) de valve constitue un passage dont l'axe (14) est situé en oblique selon un angle (a) par rapport à !a normale à l'ouverture d'entrée (15) de la bague (10) de valve, l'angle (a) de la position oblique étant situé dans un plan perpendiculaire à l'axe des tourillons (16) de telle manière que l'axe (14) du passage soit à peu près parallèle au corps de fermeture (12) ouvert.

4. Prothèse de valve cardiaque selon les revendications 2 et 3, caractérisée en ce que les trous longitudinaux sont parallèles à l'axe (14) du passage.

5. Prothèse de valve cardiaque selon l'une des revendications 1 à 4, caractérisée en ce que le corps de fermeture (12) présente, sur sa face située en aval, des surfaces de butée (21), adjacentes aux tourillons (16), qui coopèrent avec des surfaces de butée (20) de la bague (10) de valve et qui limitent le mouvement d'ouverture du corps de fermeture (12) à un angle inférieur à 90°.

6. Prothèse de valve cardiaque selon l'une des revendications 1 à 5, caractérisée en ce que les tourillons (16) ne sont pas en saillie au-delà du contour extérieur de la bague (10) de valve et présentent des faces frontales obliques (16a) qui reculent vers l'amont dans la position d'ouverture du corps de fermeture (12).

7. Prothèse de valve cardiaque selon la revendication 6, caractérisée en ce qu' il est prévu, sur le côté intérieur de la bague (10) de valve, des surfaces de butée (19), adjacentes aux trous traversants (17), qui reculent vers l'extérieur dans la direction d'écoulement et qui sont adjacentes à des surfaces de butée du corps de fermeture (12).

8. Prothèse de valve cardiaque selon l'une des revendications 1 à 7, caractérisée en ce que le corps de fermeture (12) est d'une ossature incurvée en forme de S et est en outre bombé en direction transversale.

FIG. 1

**FIG. 2**

**FIG. 3**

FIG. 4

FIG. 5

FIG. 6